(19) Europäisches Patentamt
European Patent Office
Office européen des brevets

(11) Veröffentlichungsnummer: **0 129 904**
**B1**

(12) **EUROPÄISCHE PATENTSCHRIFT**

(45) Veröffentlichungstag der Patentschrift: **18.01.89**

(51) Int. Cl.[4]: **C 07 D 265/30**

(21) Anmeldenummer: **84107282.0**

(22) Anmeldetag: **25.06.84**

(54) **Verfahren zur Herstellung von cis-2,6-Dimethylmorpholin.**

(30) Priorität: **25.06.83 DE 3322939**

(43) Veröffentlichungstag der Anmeldung: **02.01.85 Patentblatt 85/1**

(45) Bekanntmachung des Hinweises auf die Patenterteilung: **18.01.89 Patentblatt 89/3**

(84) Benannte Vertragsstaaten: **BE CH DE FR GB IT LI NL**

(56) Entgegenhaltungen:
**EP-A- 0 007 520**
**EP-A- 0 026 367**
**US-A- 3 083 202**

(73) Patentinhaber: **BASF Aktiengesellschaft, Carl-Bosch-Strasse 38, D-6700 Ludwigshafen (DE)**

(72) Erfinder: **Goetz, Norbert, Dr., Schoefferstrasse 25, D-6520 Worms 1 (DE)**
Erfinder: **Hupfer, Leopold, Dr., Waltershoehe 3, D-6701 Friedelsheim (DE)**
Erfinder: **Franzischka, Wolfgang, Dr., Franz-Lind-Strasse 1, D-6713 Freinsheim (DE)**

**EP 0 129 904 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft ein Verfahren zur Herstellung von cis-2,6-Dimethylmorpholin aus schwefelhaltigem trans-2,6-Dimethylmorpholin durch Isomerisierung an Platin-, Rithenium- oder Rhodiumkatalysatoren in Gegenwart von Wasserstoff.

Es ist bekannt, dass man in einem Gemisch aus cis- und trans-2,6-Dimethylmorpholin durch Erhitzen dieses Gemisches mit konzentrierter oder rauchender Schwefelsäure bei Temperaturen zwischen 185 und 220 °C den Anteil der cis-Verbindung erhöhen kann (US-A-3 083 202). Weiterhin ist es aus EP-A-7 520 und DE-A-2 938 698 bekannt, dass sich diese cis/trans-Umlagerung auch an Hydrierkatalysatoren durchführen lässt. Allerdings werden nur mit Palladium-Katalysatoren oder Palladium-Zink(Cadmium, Mangan)-Katalysatoren Selektivitäten über 90% bei gleichzeitig guten Isomerisierungsraten erhalten. Nickel- und Cobaltkatalysatoren liefern wesentlich schlechtere Ergebnisse.

Es wurde nun gefunden, dass man cis-2,6-Dimethylmorpholin der Formel (I)

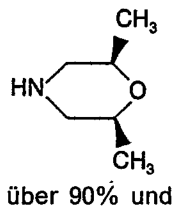

I

mit Selektivitäten über 90% und hohen Isomerisierungsraten in einfacher Weise erhält, wenn man schwefelhaltiges trans-2,6-Dimethylmorpholin der Formel (II)

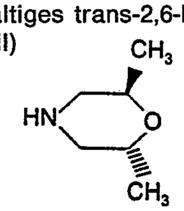

II

in Gegenwart von Wasserstoff an einem Platin-. Ruthenium- oder Rhodium-haltigen Katalysator, der 0,05 bis 15 Gew.% des Metalls auf einem Träger enthält, bei Temperaturen von 150 bis 250 °C, bei einem Druck von Normaldruck bis 200 bar umsetzt.

Ein schwefelhaltiges trans-2,6-Dimethylmorpholin enthält beispielsweise 1 bis 50, vorzugsweise 4 bis 12 ppm Schwefel.

Die Herstellung von 2,6-Dimethylmorpholin im technischen Massstab erfolgt bekanntlich durch Cyclisierung von Diisopropanolamin mit konzentrierter Schwefelsäure oder Oleum bei höheren Temperaturen (US-A-3 083 202, Houben-Weyl, «Methoden der organischen Chemie», 4. Auflage, Georg Thieme Verlag, Stuttgart 1966, Band 6/4, Seiten 510 ff). Da sich mit konzentrierter Schwefelsäure in Anwesenheit von organischem Material bei höheren Reaktionstemperaturen Redoxvorgänge abspielen (H: Remy, Lehrbuch der anorg. Chemie, 13. Auflage, Akademische Verlagsgesellschaft Geest und Portig KG, Leipzig 1970, Band I, Seiten 861, 921 bis 922), bilden sich im Verlaufe der Reaktion Schwefelverbindungen niedriger Wertigkeitsstufe, die sich – auch bei aufwendiger Reinigung – nicht vollständig vom Endprodukt abtrennen lassen. Das hat zur Folge, dass selbst durch Destillation gereinigtes technisches 2,6-Dimethylmorpholin (Isomerengemisch) – in Abhängigkeit vom Trennaufwand – beispielsweise von 1 bis 50, insbesondere von 2 bis 20 ppm Schwefel enthält. Es hat sich gezeigt, dass Palladiumkatalysatoren – wie sie in EP-A-7 520 und DE-A-2 938 698 beschrieben werden – durch Verunreinigungen von Schwefelverbindungen unwirksam werden und die Verwendung von technischem, schwefelhaltigem 2,6-Dimethylmorpholin zur Isomerisierung mit Palladiumkatalysatoren einen raschen Abfall der Wirksamkeit der Katalysatoren zur Folge hat (siehe Vergleichsversuch 1b). Im Gegensatz dazu werden die erfindungsgemäss verwendeten Platin-, Ruthenium- oder Rhodium-haltigen Katalysatoren in ihrer Wirksamkeit nicht beeinträchtigt. Durch Verunreinigungen im technischen Dimethylmorpholin beispielsweise im Bereich von 1 bis 50, insbesondere von 2 bis 20 ppm Schwefel tritt keine Beeinträchtigung der Katalysatorwirksamkeit ein. Gleichzeitig liegen, bei hohen Isomerisierungsraten, die Selektivitäten über 90%.

Das erfindungsgemässe Verfahren kann sowohl kontinuierlich als auch diskontinuierlich durchgeführt werden. In der Regel werden Temperaturen von 150 bis 250 °C und Drucke von Normaldruck bis 200 bar, insbesondere von 1 bis 50 bar, angewendet. Es kann lösungsmittelfrei oder in Gegenwart von Lösungsmitteln, die unter den Reaktionsbedingungen inert sind, gearbeitet werden.

Als Lösungsmittel kommen beispielsweise in Frage: Cyclohexan, Cyclopentan, Methylcyclopentan, Hexan, Heptan, Cyclohexylmethylether, Tetrahydrofuran, Dioxan, Methylglykol, 1,2-Dimethyloxyethan, N-Methylpiperidin, N-Methylmorpholin, N-Methylpyrrolidin.

Der Katalysator enthält 0,05 bis 15 Gew.% Platin, Ruthenium oder Rhodium auf einem Träger, wie beispielsweise Aktivkohle, Kieselgel, Aluminiumsilikat oder vorzugsweise Aluminiumoxid, Spinelle des Aluminiums oder Mischungen aus Aluminiumoxid und basischen Metalloxiden. Bevorzugte basische Metalloxide sind Calciumoxid und Magnesiumoxid. Die Herstellung der Katalysatoren erfolgt durch Tränken des Trägermaterials mit Lösungen von z.B. Nitraten, Chloriden, Formiaten oder Oxalaten der Edelmetalle. Durch anschliessende thermische Behandlung, die gewöhnlich zwischen 400 und 600 °C durchgeführt wird, erfolgt ihre Überführung in die Oxidform. Soll bei Aluminiumoxidträgern mit geeigneten Metallen (Mg, Zn, Co, Mn, Ni, Li) eine Spinellbildung des Trägermaterials erzielt werden, dann muss nach der Tränkung des Aluminiumoxids mit Lösungen der Metalle für die Spinellbildung auf eine Temperatur von 900 bis 1300 °C erhitzt werden (siehe Ullmanns Encyklopädie der techn. Chemie, 3. Auflage (1955), Band 6, Seiten 242–244; Gmelin, System-Nr. 35, Al, Tl 1934–1935, Seiten 26–28) und anschliessend in üblicher Weise das Trägermaterial mit den Lösungen der Edelmetalle

getränkt und weiterbehandelt werden wie oben beschrieben.

Man verwendet den Katalysator beispielsweise in Form von Strängen mit einem Durchmesser von 3 mm und einer Länge von 10 mm oder als Pulver, je nach dem vorgesehenen Verwendungszweck.

Nach dem erfindungsgemässen Verfahren wird trans-2,6-Dimethylmorpholin bei gleichzeitig sehr guter Gesamtausbeute an 2,6-Dimethylmorpholin weitgehend in das cis-2,6-Dimethylmorpholin umgelagert. Vorteilhaft ist dabei auch die lange Lebensdauer der verwendeten Edelmetallkatalysatoren; so ist bei schwefelhaltigem Einsatzprodukt und bei einer Dauerbeanspruchung von über 1000 Betriebsstunden kein Aktivitätsabfall des Katalysators zu verzeichnen.

Das nach dem Verfahren der Erfindung hergestellte cis-2,6-Dimethylmorpholin findet als Zwischenprodukt für die Herstellung von Wirkstoffen in Pflanzenschutzmitteln Verwendung (DE-A-2 656 747, 2 752 096, 2 752 135).

Die Messung des in den folgenden Beispielen angegebenen Druckes – bei Destillationen unter vermindertem Druck – erfolgte in mbar.

Beispiel 1a

In ein zylindrisches Reaktionsrohr mit einem Volumen von 1 Liter wird ein Katalysator in Form von Strängen (4 mm Durchmesser, 10 mm Länge), der 0,5 Gew.% Platin auf einer Mischung aus 19,4 Gew.% Magnesiumoxid und 80,6 Gew.% Aluminiumoxid enthält, eingefüllt und auf 190 °C erhitzt. Über dieses Katalysatorbett werden stündlich 100 g gasförmiges trans-2,6-Dimethylmorpholin, das 8 ppm Schwefel in Form von schwefelhaltigen Verunreinigungen enthält, und im Gleichstrom 100 Normalliter Wasserstoff hindurchgeleitet. Das Reaktionsprodukt wird, sobald es den Reaktor verlässt, abgekühlt. Man erhält pro Stunde 100 g Umsetzungsprodukt.

Die gaschromatographische Analyse des Umsetzungsproduktes zeigt einen Gehalt an gesamtem 2,6-Dimethylmorpholin von 98,2%, das Isomerenverhältnis beträgt: 88% cis-2,6-Dimethylmorpholin/12% trans-2,6-Dimethylmorpholin. Diese Werte konnten über eine Dauer von 1000 Betriebsstunden aufrechterhalten werden.

Eine Fraktionierung von 800 g des Rohproduktes über eine Füllkörperkolonne mit 65 theoretischen Böden liefert 637 g reines cis-2,6-Dimethylmorpholin, $Kp_{133}$ = 80–81 °C. Das entspricht einer Ausbeute an cis-2,6-Dimethylmorpholin von 79,8%. Weiterhin fallen 145 g einer Fraktion an, die überwiegend aus trans-2,6-Dimethylmorpholin ($Kp_{133}$ = 87–90 °C) besteht und wieder in die Isomerisierung zurückgeführt werden kann. An hochsiedenden Produkten (Destillationsrückstand) werden 18 g erhalten. Die Selektivität der Reaktion beträgt danach 97,9%.

Beispiel 1b (Vergleichsversuch)

Man arbeitet analog Beispiel 1a bei einer Reaktionstemperatur von 220 °C und verwendet dasselbe schwefelhaltige trans-2,6-Dimethylmorpholin, aber einen Katalysator, der 0,5 Gew.% Palladium auf einer Mischung aus 19,4 Gew.% Magnesiumoxid und 80,6 Gew.% Aluminiumoxid enthält. Die übrigen Reaktionsbedingungen sind identisch mit denen in Beispiel 1a.

Nach gaschromatographischer Analyse ergibt sich für das Umsetzungsprodukt in Abhängigkeit von der Zeitdauer folgende Isomerenzusammensetzung:

Nach einer Stunde: 83% cis- und 17% trans-2,6-Dimethylmorpholin

nach zehn Stunden: 77% cis- und 23% trans-2,6-Dimethylmorpholin

nach dreissig Stunden: 41% cis- und 59% trans-2,6-Dimethylmorpholin.

Beispiel 2

Man verfährt analog Beispiel 1a, verwendet aber einen Katalysator, der 0,2 Gew.% Platin auf einem Magnesium-Aluminiumspinellträger enthält, bei einer Reaktionstemperatur von 200 °C. Man erhält unter diesen Bedingungen – bei guten Katalysatorstandzeiten – folgende Ergebnisse:

Das Isomerenverhältnis beträgt 86% cis-/14% trans-2,6-Dimethylmorpholin bei einem Gesamtgehalt an 2,6-Dimethylmorpholin im Reaktionsprodukt von 96,4%.

Beispiel 3

Man verfährt analog Beispiel 1a, verwendet aber einen Katalysator, der 0,5 Gew.% Platin auf einem Lithium-Aluminiumspinellträger enthält, bei einer Reaktionstemperatur von 180 °C.

Man erhält unter diesen Bedingungen – bei guten Katalysatorstandzeiten – folgende Ergebnisse:

Das Isomerenverhältnis beträgt 86% cis-/14% trans-2,6-Dimethylmorpholin bei einem Gesamtgehalt an 2,6-Dimethylmorpholin im Reaktionsprodukt von 97,3%.

Beispiel 4

Man verfährt analog Beispiel 1a, verwendet aber einen Katalysator, der 0,5 Gew.% Platin auf gamma-Aluminiumoxid enthält, bei einer Reaktionstemperatur von 185 °C.

Man erhält unter diesen Bedingungen – bei guten Katalysatorstandzeiten – folgende Ergebnisse:

Das Isomerenverhältnis beträgt 88% cis-/12% trans-2,6-Dimethylmorpholin bei einem Gesamtgehalt an 2,6-Dimethylmorpholin von 95,8%.

Beispiel 5a

In einen Rollautoklaven mit einem Inhalt von 3 l wurden 1500 g trans-2,6-Dimethylmorpholin eingefüllt, die 12 ppm Schwefel in Form von schwefelhaltigen Verunreinigungen enthielten. Weiterhin wurden 20 g Katalysator mit der Zusammensetzung 5 Gew.% Ruthenium auf gamma-Aluminiumoxid zugegeben. Der Autoklav wurde druckdicht verschlossen, mit Stickstoff gespült und anschliessend wurden 10 bar Wasserstoff aufgepresst. Danach wurde der Autoklav auf 210 °C erhitzt und 10 Stunden lang auf dieser Temperatur gehalten. Anschliessend wurde abgekühlt, der Katalysator abfiltriert und die Zusammensetzung des Filtrates

durch eine gaschromatographische Analyse bestimmt.

Hierbei wurden folgende Ergebnisse erhalten: Das Isomerenverhältnis betrug 88% cis-/12% trans-2,6-Dimethylmorpholin bei einem Gesamtgehalt von 94,6% 2,6-Dimethylmorpholin im Reaktionsprodukt. Der Katalysator konnte – ohne Aktivitätsverlust – in zehn weiteren Versuchen eingesetzt werden.

Beispiel 5b (Vergleichsversuch)

Man arbeitet analog Beispiel 5a bei einer Reaktionstemperatur von 230 °C und verwendet das gleiche schwefelhaltige trans-2,6-Dimethylmorpholin, aber einen Katalysator, der 10,0 Gew.% Palladium, 0,11 Gew.% Zink und 0,1 Gew.% Cadmium auf Aluminiumoxid enthält. Die übrigen Reaktionsbedingungen sind identisch mit denen in Beispiel 5a.

Für das Reaktionsprodukt ergibt sich nach gaschromatographischer Analyse folgende Isomerenzusammensetzung:
Nach einmaliger Verwendung des Katalysators: 86% cis/14% trans-2,6-Dimethylmorpholin,
nach zweimaliger Verwendung des Katalysators: 32% cis-/68% trans-2,6-Dimethylmorpholin,
nach dreimaliger Verwendung des Katalysators: keine Isomerisierung.

Beispiel 6

Man verfährt analog Beispiel 5, verwendet aber einen Katalysator, der 1,8 Gew.% Ruthenium auf einem Zink-Aluminiumspinellträger enthält, und arbeitet bei einer Reaktionstemperatur von 230 °C. Unter diesen Bedingungen wurden folgende Ergebnisse erhalten:

Das Isomerenverhältnis betrug 84% cis-/16% trans-2,6-Dimethylmorpholin bei einem Gesamtgehalt von 93,7% 2,6-Dimethylmorpholin im Reaktionsprodukt.

Beispiel 7

Man verfährt analog Beispiel 5, verwendet aber einen Katalysator, der 5,0 Gew.% Rhodium auf gamma-Aluminiumoxid enthält und arbeitet bei einer Reaktionstemperatur von 200 °C.

Unter diesen Bedingungen wurden folgende Ergebnisse erhalten:Das Isomerenverhältnis betrug 88% cis-/12% trans-2,6-Dimethylmorpholin bei einem Gesamtgehalt von 95,4% 2,6-Dimethylmorpholin im Reaktionsprodukt.

Beispiel 8

Man verfährt analog Beispiel 5, verwendet aber einen Katalysator, der 1,8 Gew.% Rhodium auf einer Mischung aus 19,4 Gew.% Calciumoxid und 80,6 Gew.% Aluminiumoxid als Träger enthält und arbeitet bei einer Reaktionstemperatur von 220 °C.

Unter diesen Bedingungen wurden folgende Ergebnisse erhalten:

Das Isomerengemisch betrug 84% cis-/16% trans-2,6-Dimethylmorpholin bei einem Gesamtgehalt von 94,3% 2,6-Dimethylmorpholin im Reaktionsprodukt.

## Patentansprüche

1. Verfahren zur Herstellung von cis-2,6-Dimethylmorpholin durch Isomerisierung von trans-2,6-Dimethylmorpholin in Gegenwart von Wasserstoff und einem Katalysator, der ein Metall der Gruppe VIII des Periodensystems der Elemente enthält, bei einer Temperatur zwischen 150 und 250 °C und bei einem Druck zwischen Normaldruck und 200 bar, dadurch gekennzeichnet, dass man schwefelhaltiges trans-2,6-Dimethylmorpholin in Gegenwart von einem Platin-, Ruthenium- oder Rhodium-haltigen Katalysator, der 0,05 bis 15 Gew.% des Metalls auf einem Träger, enthält, umsetzt.

2. Verfahren gemäss Anspruch 1, dadurch gekennzeichnet, dass man als Trägermaterial für den Katalysator Aluminiumspinelle verwendet.

3. Verfahren gemäss Anspruch 1, dadurch gekennzeichnet, dass man einen Katalysator verwendet, der Calciumoxid oder Magnesiumoxid im Träger enthält.

## Claims

1. A process for preparing cis-2,6-dimethylmorpholine by isomerizing trans-2,6-dimethylmorpholine in the presence of hydrogen and a catalyst containing a metal of group VIII of the periodic table of the elements at from 150 to 250 °C and a pressure of from atmospheric pressure to 200 bar, which comprises reacting sulfur-containing trans-2,6-dimethylmorpholine in the presence of a platinum-, ruthenium- or rhodium-containing catalyst containing from 0.05 to 15% by weight of the metal on a carrier.

2. A process as claimed in claim 1, wherein the carrier material used for the catalyst is aluminium spinel.

3. A process as claimed in claim 1, wherein the catalyst used contains calcium oxide or magnesium oxide in the carrier.

## Revendications

1. Procédé de préparation de cis-2,6-diméthylmorpholine par Isomérisation de trans-2,6-diméthylmorpholine en présence d'hydrogène et d'un catalyseur qui contient un métal du groupe VIII du système périodique des éléments, à une température comprise entre 150 et 250 °C et à une pression comprise entre la pression normale et 200 bar, caractérisé par le fait que l'on fait réagir de la trans-2,6-diméthylmorpholine à teneur en soufre en présence d'un catalyseur à teneur en platine, ruthenium ou rhodium, qui contient 0,05 à 15% en poids du métal sur support.

2. Procédé selon la revendication 1, caractérisé par le fait que l'on utilise, comme matériau support pour le catalyseur, du spinelle d'aluminium.

3. Procédé selon la revendication 1, caractérisé par le fait que l'on utilise un catalyseur qui contient dans le support de l'oxyde de calcium ou de l'oxyde de magnésium.